**Europäisches Patentamt**

**European Patent Office**

**Office européen des brevets**

(19)

(11) Veröffentlichungsnummer: **0 051 718**
**B1**

(12)

# EUROPÄISCHE PATENTSCHRIFT

(45) Veröffentlichungstag der Patentschrift:
**14.03.84**

(51) Int. Cl.³: **A 61 M 25/00**

(21) Anmeldenummer: **81106479.9**

(22) Anmeldetag: **20.08.81**

(54) Einführungsvorrichtung für Katheter.

(30) Priorität: **08.11.80 DE 3042229**

(43) Veröffentlichungstag der Anmeldung:
**19.05.82 Patentblatt 82/20**

(45) Bekanntmachung des Hinweises auf die Patenterteilung:
**14.03.84 Patentblatt 84/11**

(84) Benannte Vertragsstaaten:
**AT BE CH DE FR GB IT LI LU NL SE**

(56) Entgegenhaltungen:
**DE - A - 2 501 428**
**DE - A - 2 516 219**
**DE - B - 2 456 980**
**GB - A - 2 019 219**
**US - A - 3 557 778**

(73) Patentinhaber: **INTERMEDICAT GMBH,
Gerliswiistrasse 43, CH-6020 Emmenbrücke (CH)**

(72) Erfinder: **Schacht, Bodo, Weihersgrund 1,
D-3509 Malsfeld (DE)**

(74) Vertreter: **von Kreisler, Alek, Dipl.-Chem. et al,
Deichmannhaus am Hauptbahnhof, D-5000 Köln 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

## Einführungsvorrichtung für Katheter

Die Erfindung betrifft eine Einführungsvorrichtung für Katheter, mit einem Gehäusekörper, der einen längslaufenden durchgehenden, mit einer Kanüle zu verbindenden Kanal aufweist, und mit zwei, am rückwärtigen Ende des Gehäusekörpers hintereinander angeordneten, jeweils aus elastischem Material bestehenden Dichtungselementen, die aufweitbare Öffnungen für den Durchtritt langgestreckter Gegenstände aufweisen.

Zur Einführung von Kathetern in Blutgefäße ist es bekannt, zunächst eine aus Metall oder Kunststoff bestehende Kanüle in das Lumen des Blutgefäßes einzuführen und durch diese Passage bis zur benötigten Länge vorzuschieben. In die Kanüle wird anschließend der Katheter eingeschoben. Danach wird die Kanüle zurückgezogen, während der Katheter im Gefäß verbleibt. Durch den zwischen Katheter und Einführungskanüle bestehenden Spalt kann Blut aus dem Blutgefäß nach außen fließen. Durch geeignete Maßnahmen muß daher dafür gesorgt werden, daß dieser Blutverlust in Grenzen gehalten wird. Dies ist bei dem relativ niedrigen venösen Blutdruck ohne größere Schwierigkeiten möglich. Bei arterieller Punktion ist die Abdichtung des Spaltes zur Vermeidung von Blutverlusten und zur Verhinderung von Kontaminationen jedoch erheblich schwieriger.

Bei einer bekannten Einführungsvorrichtung der eingangs genannten Art (US-A-4 000 739) sind in einem Gehäuse zwei miteinander kombinierte Dichtungselemente vorgesehen, von denen das erste aus einer Scheibe besteht, die einen zentralen Y-förmigen Einschnitt aufweist, durch den der Katheter unter Aufspreizung des Dichtungselementes hindurchgeschoben werden kann. Das zweite Dichtungselement ist eine ebene Scheibe mit einem zentralen Loch, die das erste Dichtungselement abstützt. Bei einem in dem Gehäusekörper auftretenden Druck wird das erste Dichtungselement fest gegen das hinter ihm angeordnete zweite Dichtungselement gedrückt. Auf diese Weise wird eine Katheterschleuse gebildet, die sowohl bei nicht eingeführtem Katheter als auch bei eingeführtem Katheter, sowie in der Einführungsphase, das Kanülenende luft- und flüssigkeitsdicht abschließt.

Um die Einführungsvorrichtung für Katheter mit unterschiedlichen Stärken benutzen zu können, müssen verschiedene Dichtungsscheiben verfügbar sein, die als zweite Dichtungselemente in das Gehäuse eingesetzt werden und deren Bohrungsdurchmesser jeweils auf den Durchmesser des zu verwendenden Katheters abgestimmt ist. Diese unterschiedlichen Dichtungsscheiben führen beim Gebrauch leicht zu Irrtümern und Verwechslungen, die erhebliche Komplikationen zur Folge haben können. Ein weiterer Nachteil des vorbestimmten Bohrungsdurchmessers tritt dann auf, wenn im Zuge einer einzigen Anwendung der Einführungsvorrichtung zwei im Durchmesser sehr unterschiedliche Elemente unter Aufrechterhaltung der Dichtungseigenschaften durch die Dichtungselemente hindurchgeschoben werden müssen. Bei bestimmten Anwendungsformen wird in einem ersten Arbeitsschritt z. B. eine Führungsspirale mit einem Durchmesser von 0,6 mm und in einem zweiten Arbeitsschritt ein Katheter mit einem Außendurchmesser von 1,8 mm durch die Einführungsvorrichtung hindurchgeführt. Für einen derartig großen Durchmesserbereich ist das zweite Dichtungselement ungeeignet.

Aber auch das vor dem zweiten Dichtungselement angeordnete erste Dichtungselement mit symmetrischem Y-Einschnitt bewirkt keine Abdichtung in dem erforderlichen Maße. Aus geometrischen Gründen können die durch den Y-Einschnitt gebildeten Dreieckflächen, deren Spitzen nach vorne ragen, den Umfang des Katheters nicht vollständig abdichtend bedecken, so daß an den Spitzen des Einschnittes Undichtigkeiten entstehen. Darüber hinaus nimmt eine Dichtungsscheibe, die durch Schlitze in dreieckige Lappen unterteilt ist, nach einer Aufbiegung der Lappen nicht mehr genau ihren ursprünglichen Zustand an, bei dem die Schlitze wieder vollständig geschlossen wären. Bei der Zurückbewegung in die ursprüngliche Ausgangslage behindern sich die Lappen gegenseitig. Die Rückstellkraft des gummielastischen Materials reicht nicht aus, um diese Widerstände zu überwinden.

Der Erfindung liegt die Aufgabe zugrunde, eine Einführungsvorrichtung der eingangs genannten Art dahingehend zu verbessern, daß ohne Auswechslung der Dichtungselemente Teile mit unterschiedlichen Durchmessern abgedichtet werden können.

Zur Lösung dieser Aufgabe ist erfindungsgemäß vorgesehen, daß das erste Dichtungselement aus einem im Ruhezustand rohrförmigen Teil besteht, dessen vorderer Abschnitt frei in den Kanal des Gehäusekörpers hinein axial vorsteht und an seiner Stirnseite zwei radial gegeneinander drückende Dichtlippen aufweist, und daß das zweite Dichtungselement aus einer zwischen dem rückwärtigen Ende des Gehäusekörpers und dem ersten Dichtungselement angeordneten Scheibe besteht, die eine ohne Materialentnahme hergestellte aufweitbare Öffnung aufweist.

Beim Einschieben eines Katheters oder eines anderen langgestreckten Elementes in die Einführungsvorrichtung wird zunächst die in einem dünnen membranartigen Wandbereich des zweiten Dichtungselements liegende perforierte Durchtrittsstelle aufgeweitet. Die Wandstärke und das Material des zweiten Dichtungselementes sind so gewählt, daß Katheter und Führungsdrähte unterschiedlicher Durchmesser mit zumutbarem Kraftaufwand durch die Perforationsstelle hindurchgeschoben werden können, und daß gleichzeitig die Scheibe wegen ihrer hohen Flexibilität radial abdichtend an dem Umfang des durchgeführten Gegenstandes fest anliegt. Der Rand der Scheibe kann dicker ausgebildet sein als der membranartige Mittelbereich.

Das erste Dichtungselement, das sich unmittelbar an das zweite Dichtungselement anschließt, ist ein kegeliges, teilkegeliges oder zylindrisches Hohlteil, das in seinem vorderen Bereich durch ein Längsschnitt geteilt ist und dadurch zwei Lippen bildet. Beim Hindurchschieben eines Gegenstandes spreizen sich die Lippen auseinander. Wird der Gegenstand anschließend zurückgezogen, legen sich infolge der Rückstellkräfte die Lippen wieder mit ihren Schnittkanten gegeneinander. Wenn im Innern des Gehäusekörpers ein Druck herrscht, wirkt dieser auf die Außenfläche des rohrförmigen Teiles ein, wodurch die Dichtlippen verstärkt gegeneinander gedrückt bzw. gegen die Umfangswand des durchgeführten Gegenstandes gepreßt weden. Die Dichtlippen bewegen sich hierbei ausschließlich radial zur Achse des ersten Dichtungselementes.

Im Zusammenwirken des zweiten Dichtungselementes der aus dem Ende eines im wesentlichen Teiles gebildeten Dichtlippen und eines weiteren Dichtungselementes mit einer ohne Materialbeseitigung perforierten elastischen Scheibe oder Membran wird eine den gestellten Anforderungen entsprechende Abdichtung bei gleichzeitiger Variabilität in den Durchmesser der durchgeführten Gegenstände erreicht.

Im folgen wird unter Bezugnahme auf die Figuren ein Ausführungsbeispiel der Erfindung näher erläutert. Es zeigt

Fig. 1 ein Längsschnitt durch die Einführungsvorrichtung und

Fig. 2 eine Schnittzeichnung der wesentlichen Teile der Einführungsvorrichtung im auseinandergenommenen Zustand.

Die Einführungsvorrichtung 1 weist einen im wesentlichen rohrförmigen Gehäusekörper 2 auf, dessen eines Ende mit einer Kappe 3 bedeckt ist, die eine mittig angeordnete, sich kegelförmig nach außen erweiternde Bohrung 4 in ihrer Stirnseite aufweist. Die Kappe 3 kann durch Klebung, Schweißung oder mit einer einrastenden Schnappverbindung an dem Gehäusekörper 2 befestigt sein.

Der Gehäusekörper 2 weist eine längslaufende Bohrung 5 auf, die sich durch mehrere stufenförmige Absätze, die jeweils eine Ringschulter 6, 7, 8 bilden, zu dem rückwärtigen; durch die Kappe 3 abgedeckten Ende hin erweitert.

Gegen die Ringschulter 6 des Gehäusekörpers 2 ist der überstehende Rand 9 des ersten Dichtungselementes 10 gelegt. Dieses erste Dichtungselement 10 ist im wesentlichen rohrförmig ausgebildet und erstreckt sich von dem Rand 9 aus in der Bohrung 5 frei nach vorne. Das vordere Ende des Dichtungselementes 10 ist kegelförmig verjüngt, wobei die Spitze abgerundet ist. Das vordere Ende des Dichtungselementes 10 ist ferner durch einen Längsschlitz 11 in zwei symmetrische Dichtlippen 12, 13 unterteilt, die auseinandergespreizt werden können. Der Längsschlitz 11 erstreckt sich über einen wesentlichen Teil der Länge des Dichtungselementes 10, z. B. über einen Bereich in der Größenordnung von $1/3$ bis $1/2$ der Länge. Das Dichtungselement 10 wirkt mit seinen Dichtlippen 12, 13 als Rückstromsperre. Der in seinem Inneren gebildete Kanal 14 ist so ausgelegt, daß rohr- oder stabförmige Gegenstände mit dem größten benötigten Durchmesser ohne Wandreibung durch den zylindrischen Teil des Dichtungselementes hindurchgeführt werden können.

Der Rand 9 des Dichtungselementes 10 hat eine solche Stärke, daß er den Bereich zwischen den beiden Ringschultern 6 und 7 im wesentlichen ausfüllt.

Gegen die Ringschulter 7 ist der Rand 15 des zweiten Dichtungselementes 16 gelegt. Dieses zweite Dichtungselement 16 ist als Scheibe ausgebildet, die einen dünnen membranartigen Mittelbereich 17 aufweist, welcher von dem dick ausgebildeten Rand 15 umgeben ist. In der Mitte des membranartigen Bereichs 17 ist die ohne Materialentnahme hergestellte Perforation oder Öffnung 18 angeordnet. Der membranartige Bereich 17 wirkt als ausgleichendes und abdichtendes Element für die unterschiedliche Durchmesser aufweisenden durchgeführten Katheter und Führungselemente.

Gegen die rückwärtige Ringschulter 8 ist eine Halteplatte 19 gelegt, die gegen die rückwärtige Stirnseite des Randes 15 des zweiten Dichtungselementes 10 drückt und die Einbauhöhen der Dichtungselemente 10 und 16 ausgleicht. Die Halteplatte 19 weist eine zentrische Bohrung 20 auf, die dem größten einzuführenden Durchmesser angepaßt ist und unterhalb der Öffnung 4 der Kappe 3 liegt.

An dem vorderen Ende des Gehäusekörpers 2 befindet sich einaxial abstehender Kegelansatz 21, auf den ein Anschlußstück 22, das eine kegelförmige Bohrung 23 aufweist, abdichtend aufschiebbar ist. In die Bohrung 23 mündet eine an dem Anschlußstück 22 befestigte Kanüle 24 ein.

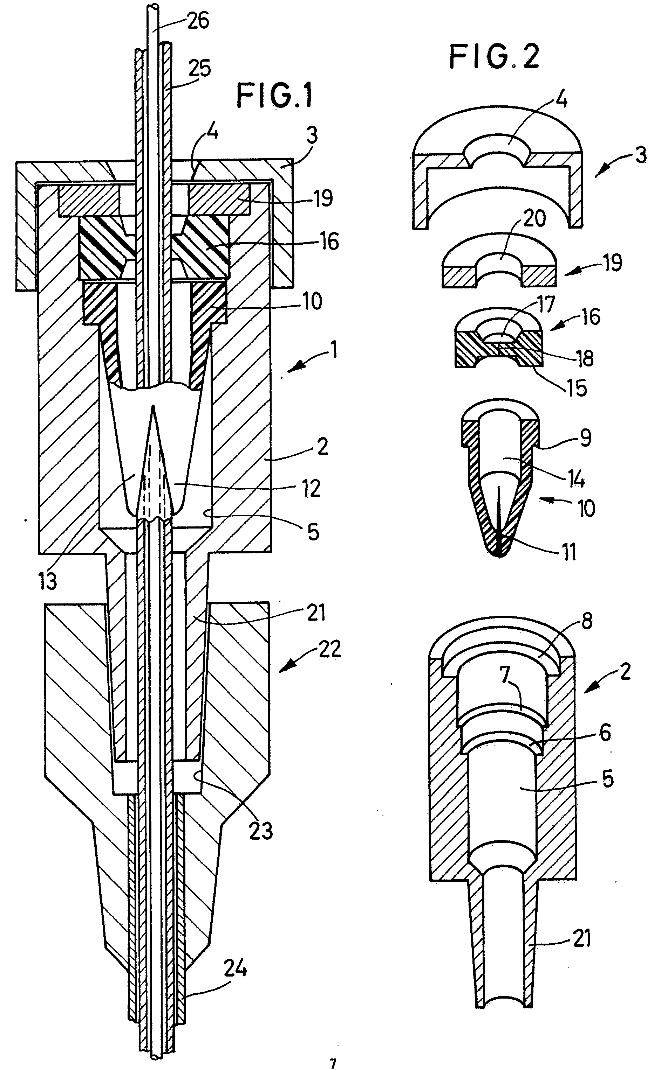

Bei der Benutzung der dargestellten Einführungsvorrichtung erfolgt zunächst eine Gefäßpunktion mit einer (nicht dargestellten) Punktionskanüle. Durch die Punktionskanüle hindurch wird der Führungsdraht 26 in das Blutgefäß eingeführt und zum Untersuchungsort vorgeschoben. Anschließend wird die Punktionskanüle entfernt. Dann wird der Dilatator 25 zusammen mit der ihn, gemäß Fig. 1, umgebenden Kanüle 24 über den Führungsdraht aufgeschoben und in das Blutgefäß gebracht. Durch den Spalt zwischen der Kanüle 24 und dem Dilatator 25 hindurch kann Blut ausfließen. Um dies zu verhindern, sind in dem Gehäusekörper 2 die Dichtungselemente 10 und 16 angeordnet. Der im Inneren der Bohrung 5 entstehende Blutdruck drückt die Dichtlippen 12 seitlich fest gegen den Dilatator 25, so daß aus dem Gehäusekörper 2 kein Blut herausdringen kann.

Anschließend wird der Dilatator 25 zusammen mit dem Führungsdraht 26 durch Zurückziehen entfernt. Dabei legen sich die Dichtlippen 12, 13 des ersten Dichtungselementes 10 mit ihren Dichtflächen gegeneinander und verschließen die Passage gegen ausströmendes Blut.

Wenn die Kanüle 24 auf die beschriebene Weise plaziert worden ist, dient sie als Einführungsrohr, durch das hindurch Katheter, Sonden oder andere Einrichtungen an die punktierte Stelle herangeführt werden können. Zu diesem Zweck wird der Katheter od. dgl. durch die Öffnungen des zweiten Dichtungselementes 16 und des ersten Dichtungselementes 10 hindurch in die Kanüle 24 eingeschoben. Die Öffnungen passen sich elastisch an den Durchmesser des Katheters an und bewirken eine druckdichte Abdichtung. Sie bestehen vorzugsweise aus einem vernetzten Kautschuk, z. B. auf Isopren- oder Silikonbasis. Auch thermoplastische Elastomere sind geeignet.

## Patentansprüche

1. Einführungsvorrichtung für Katheter, Sonden od. dgl., mit einem Gehäusekörper (2), der einen längslaufenden durchgehenden, mit einer Kanüle (24) zu verbindenden Kanal (5) aufweist, und mit zwei, am rückwärtigen Ende des Gehäusekörpers hintereinander angeordneten, jeweils aus elastischem Material bestehenden Dichtungselementen (10, 16), die aufweitbare Öffnungen (11, 18) für den Durchtritt langgestreckter Gegenstände (25, 26) aufweisen, dadurch gekennzeichnet, daß das erste Dichtungselement (10) aus einem im Ruhezustand rohrförmigen Teil besteht, dessen vorderer Abschnitt frei in den Kanal (5) des Gehäusekörpers (2) hinein axial vorsteht und an seiner Stirnseite zwei gegeneinander drückende Dichtlippen (12, 13) aufweist, und daß das zweite Dichtungselement (16) aus einer zwischen dem rückwärtigen Ende des Gehäusekörpers und dem ersten Dichtungselement (10) angeordneten Scheibe besteht, die eine ohne Materialentnahme hergestellte aufweitbare Öffnung (18) aufweist.

2. Einführungsvorrichtung nach Anspruch 1, dadurch gekennzeichnet, daß das erste Dichtungselement (10) zu seinem vorderen Ende hin konisch verjüngt ist.

3. Einführungsvorrichtung nach Anspruch 1 oder 2, dadurch gekennzeichnet, daß das erste Dichtungselement (10) einen über die geschlossene Stirnseite hinwegführenden, in gegenüberliegende Seitenwände hineinlaufenden Schlitz (11) aufweist.

4. Einführungsvorrichtung nach einem der Ansprüche 1 bis 3, dadurch gekennzeichnet, daß die beiden Dichtungselemente (10, 16), in ihren Randbereichen (9, 15) einander berührend, unmittelbar hintereinander angeordnet sind.

5. Einführungsvorrichtung nach einem der Ansprüche 1 bis 4, dadurch gekennzeichnet, daß das zweite Dichtungselement (16) einen membranförmigen Mittelbereich (17) aufweist, dessen Stärke geringer ist als diejenige des Randbereiches (15).

6. Einführungsvorrichtung nach einem der Ansprüche 1 bis 5, dadurch gekennzeichnet, daß im rückwärtigen Bereich des Gehäusekörpers (2) mindestens eine stufenförmige Erweiterung (6, 7) zur Festlegung der Randbereiche (9, 15) der Dichtungselemente (10, 16) vorgesehen ist.

7. Einführungsvorrichtung nach einem der vorhergehenden Ansprüche, dadurch gekennzeichnet, daß die rückwärtige Öffnung des Gehäusekörpers (2) mit einem eine Öffnung (4) aufweisenden Deckel (3) verschlossen ist, der die Randbereiche (9, 15) der Dichtungselemente (10, 16) zusammengedrückt hält.

## Claims

1. An introduction device for catheters, probes or the like, comprising a housing body (2) which has a continuous longitudinal channel (5) to be connected to a cannula (24), and comprising two sealing elements (10, 16) which are positioned in tandem on the rear end of the housing body (5), are each made of elastic material and have expandable openings (11, 18) for the passage of elongate objects (25, 26), characterised in that the first sealing element (10) consists of a part which is tubular in an idle condition, the front section of which freely projects axially into the channel (5) of the housing body (2) and has at its front two sealing lips (12, 13) which press against each other, and the second sealing element (16) consists of a disc which is positioned between the rear end of the housing body and he first sealing element (10) and has an expandable opening (18) which is produced without removing any material.

2. An introduction device according to claim 1, characterised in that the first sealing element (10) is tapered conically towards its front end.

3. An introduction device according to claim 1 or 3, characterised in that the first sealing element (10) has a slit (11) which leads away over the closed front side and runs into opposite side walls.

4. An introduction device according to one of claims 1 to 3, characterised in that the two sealing elements (10, 16) are in a direct tandem position and they contact each other in their peripheral regions (9, 15).

5. An introduction device according to one of claims 1 to 4, characterised in that the second sealing element (16) has a membrane-shaped centre region (17), the thickness of which is smaller than that of the peripheral region (15).

6. An introduction device according to one of claims 1 to 5, characterised in that at least one step-shaped widening (6, 7) is provided in the rear region of the housing body (2) to establish the peripheral regions (9, 15) of the sealing elements (10, 16).

7. An introduction device according to one of the preceding claims, characterised in that the rear opening of the housing body (2) is closed by a cover (3) which has an opening (4) and which keeps the peripheral regions (9, 15) of the sealing elements (10, 16) pressed together.

**Revendications**

1. Dispositif d'entrée pour cathéters, sondes et analogues, avec un corps de boîtier (2) qui présente un canal (5) le traversant longitudinalement, à relier à une canule (24), et avec deux éléments d'étanchéité (10, 16) en matériau élastique, disposés l'un derrière l'autre à l'extrémité arrière du corps de boîtier, que présentent des orifices expansibles (11, 18) pour le passage d'objets allongés (25, 26), lequel disositif est caractérisé en ce que le premier élément d'étanchéité (10) est constitué d'une partie tubulaire en l'état au repos, dont la section avant s'engage axialement librement dans le canal (5) du corps de boîtier (2) et montre sur sa face frontale deux lèvres d'étanchéité s'appuyant l'une contre l'autre (12, 13) et en ce que le second élément d'étanchéité (16) est constitué d'un disque inséré entre l'extrémité arrière du corps de boîtier et le premier élément d'étanchéité (10), lequel disque comporte un orifice (18) expansible, formé sans enlèvement de matériau.

2. Dispositif d'entrée selon la revendication 1, caractérisé en ce que le premier élément d'étanchéité (10) est effilé coniquement jusqu'à son extrémité antérieure.

3. Dispositif d'entrée selon l'une des revendications 1 et 2, caractérisé en ce que le premier élément d'étanchéité (10) montre une fente (11) passant sur la paroi antérieure fermée, entrant dans la paroi latérale opposée.

4. Dispositif d'entrée selon l'une quelconque des revendications 1 à 3, caractérisé en ce que les deux éléments d'étanchéité (10, 16) sont disposés directement l'un derrière l'autre, reposant l'un sur l'autre dans leurs zones marginales (9, 15).

5. Dispositif d'entrée selon l'une quelconque des revendications 1 à 4, caractérisé en ce que le second élément d'étanchéité (16) montre une zone centrale (17) en forme de membrane dont l'épaisseur est plus faible que celle de la zone marginale (15).

6. Dispositif d'entrée selon l'une quelconque des revendications 1 à 5, caractérisé en ce que dans la zone arrière du corps de boîtier (2), est prévu au moins un élargissement en forme de gradins (6, 7) pour fixer les zones marginales (9, 15) des élémentes d'étanchéité (10, 16).

7. Dispositif d'entrée selon l'une quelconque des revendications précédentes, caractérisé en ce que l'orifice arrière du corps de boîtier (2) est fermé par un couvercle (3) comportant un orifice (4), qui maintient les zones marginales (9, 15) des éléments d'étanchéité (10, 16) pressées l'une contre l'autre.

FIG.1

FIG.2